(19) Europäisches Patentamt
European Patent Office
Office européen des brevets

(11) Publication number: 0 209 330 A2

(12) EUROPEAN PATENT APPLICATION

(21) Application number: 86305335.1

(22) Date of filing: 11.07.86

(51) Int. Cl.⁴: D 06 P 1/48, D 06 P 5/22, D 06 M 15/03, D 06 P 1/651, D 01 F 1/10, C 07 H 5/06, C 07 C 39/16 // D06L3/12

(30) Priority: 12.07.85 GB 8517619
12.07.85 GB 8517620
12.07.85 GB 8517621

(43) Date of publication of application: 21.01.87 Bulletin 87/4

(84) Designated Contracting States: AT BE CH DE FR IT LI LU NL SE

(71) Applicant: Cogent Limited, Temple Court 11 Queen Victoria Street, London, EC4N 4TP (GB)

(72) Inventor: Burdett, Brian Cyril, 19 Woodland Avenue, Lymm Cheshire (GB)
Inventor: Burkinshaw, Stephen Martin, Dept. of Colour Chemistry & Dyeing, The University, Leeds Yorkshire (GB)

(74) Representative: Armitage, Ian Michael et al, MEWBURN ELLIS & CO. 2/3 Cursitor Street, London EC4A 1BQ (GB)

(54) **Methods and products for treating and colouring materials.**

(57) Methods and products are described for the treatment of textile materials for softening and colouring by dyeing or printing and of colouring some non-textile materials such as glass, ceramics, wood and leather involving a treatment with an amino sugar such as chitosan, or a ring compound having the form ring-$CH_2$-ring, where the rings can be benzene or naphthalene and have at least one OH group, or compounds of an amino sugar with such a ring compound or with an anionic compound, an amphoteric compound, a non-ionic or polar compound or a neutral electrolyte, or with an amino sugar and a cationic compound. The pretreatment gives improved results with dyeing and with reactive dyes produces washfast colouring of polyester/cotton blends, of polypropylene and polyethylene and even glass, metals and ceramics.

EP 0 209 330 A2

# METHODS AND PRODUCTS FOR TREATING AND COLOURING MATERIALS

This invention relates to methods and products for treating and colouring materials, particularly but not exclusively textile materials.

It is well known that the colouring of materials depends on the nature of the material. Certain classes of dye will colour only certain types of material. Some materials are dyed only with difficulty for example at high temperatures and pressures. Polypropylene, for instance, can only be dyed when certain dye receptors have been incorporated into the synthetic polymer during its manufacture.

Combinations of different dye classes are frequently required for the coloration of fibre blends but such dyeing processes possess inherent difficulties such as restricted colour ranges, long dyeing times and elevated dyeing temperatures.

Even with dyes that work, other agents are used to improve levelness and colour yield and, for example, wash fastness and resistance to chlorinated water, so that the sheer range of dyes and dyeing aids as well as the variety of processes and techniques is daunting.

Especially for domestic dyeing it would be highly desirable to have a simple process by which a single dyestuff would be suitable for all common textile materials, and it would also be desirable if such would operate at moderate temperatures and give good, fast colour yields in reasonable times.

For industrial dyeing, the same things would be just as desirable, although <u>any</u> improvement in yields and wash fastness and any reduction in temperatures and times would be welcome, as would the possibility of simplifying inventories by increasing the number of different materials that could be dyed by a single class of dyestuff and of reducing the number of different dyeing methods in common use.

Of great advantage to the textile industry at large and to the polypropylene and polyethylene sector in particular would be the possibility of dyeing those materials instead of having to provide colour in the melt. This would facilitate the wider usage of these materials, which otherwise have excellent textile properties.

The present invention provides methods by which any single class of dye currently available to the industry can successfully colour more materials, or

colour the materials for which it is now used to deeper shades or to the same shades using less dyestuff, or be used to the same or even better effect with lower temperatures or shorter dyeing times or both. The invention also provides methods by which at least one class of dye can be used to colour all known textile materials, even those like polypropylene and polyethylene which have hitherto been regarded as undyeable, and even other non-textile materials not normally regarded as dyeable, with generally improved results in terms of colour yield and washfastness over conventional procedures.

The invention comprises a process for preparing a material for coloration by dyeing, printing and other methods and for softening the material where appropriate comprising treating the material with a treatment compound selected from the group consisting of an amino sugar, a ring compound of the form

$$Ring - CH_2 - Ring$$

a complex of an amino sugar with such a ring compound, a complex of an amino sugar with a complexing compound selected from the group consisting of an anionic compound, an amphoteric compound, a non-ionic compound, a polar compound and a neutral electrolyte, and an amino

sugar in the presence of a cationic compound.

Chitosan is a poly-amino sugar obtained from chitin, which is a polymer of 2-deoxy-2(acetyl amino) glucose found in crustacea shells or fungi. Chitosan is prepared by deacetylation of chitin with strong alkali and is a polymer of 2-deoxy-2(amino) glucose. Its use is discussed in relation to dyeing in GB-A-1 435 229, where it is said to improve colour yields of reactive dyes on cellulosics and blends of cellulosics with polyester.

The dyeing of fibre blends, especially cotton/polyester is problematical because what will dye one component will usually not dye the other at all, or at any rate not with comparable depth of shade or wash fastness. If GB-A-1 435 229 had provided a solution to this problem it would no doubt be in very common use now. However chitosan on its own, while it will increase the dye take-up of cotton for reactive dyes will not affect the polyester, which reactive dyes will simply not colour.

GB-A-1 435 229 also discusses printing chitosan on to a fabric which is then dyed to give two tone effects, the dye take-up being greater in the printed areas. Japanese Patent Application No. 45918/1968

(Publication No. 2799/1970) on the other hand discusses the use of chitosan as a dye resist agent in batik dyeing. Neither of these methods of using chitosan appears to have become commercially significant, if indeed they have been used at all.

The present invention, however, in its various aspects, provides new methods of using chitosan and compounds that can cooperate or complex with chitosan to give significant improvements in dyeing, printing and finishing generally, even enabling hitherto difficult or impossible-to-dye fabrics to be dyed or printed to deep, permanent shades using commercially available dyes.

Although there is a wide variety of compounds which can be used with chitosan, or produce a useful complex with chitosan, it is clear that some are better than others, and in general, those of small molecular size and those which contain hydroxyl groups are found particularly beneficial.

Although chitosan of different chain lengths has been found effective, there is some indication that shorter chain lengths are more effective.

Three such compounds, which are related, have been prepared as novel synthetic tanning agents having

the formulae:-

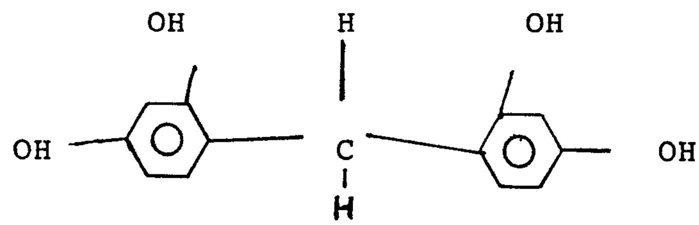

Syntan G1

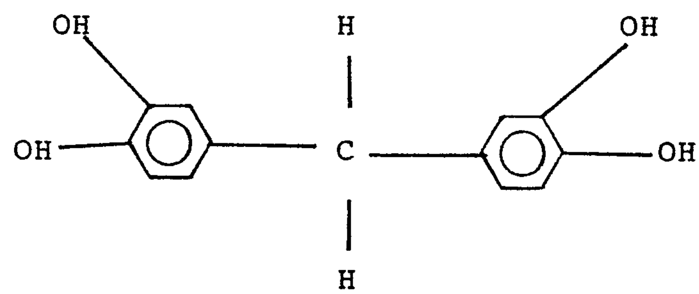

Syntan G2

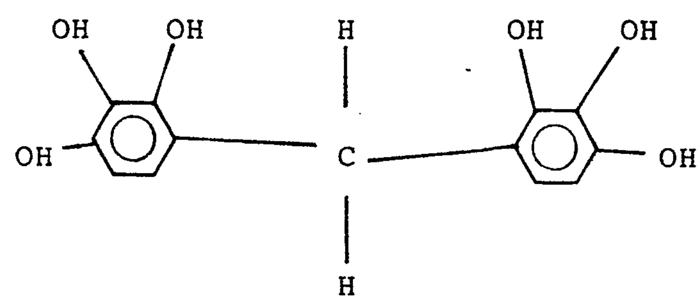

Syntan G3

These di(hydroxyphenyl) methane compounds form

complexes with chitosan which are sparingly soluble in water. Other complexing compounds useful herein may form complexes with chitosan that are soluble or insoluble in water.

The treatment may be carried out in a single step, in which the chitosan complexing agent is applied to the material, or it may be carried out by first treating the material with chitosan and then with the complexing compound, or the other way round, the complex being formed in or on the material.

The amount of chitosan applied to the material may be one or two percent by weight, as also may the amount of the complexing compound. Lesser and greater amounts, however, may also be used. At these levels, the more chitosan and/or complexing compound is applied, the better the effect in terms of dye uptake, although clearly a limit will be reached according to the type of material being treated beyond which no further increase, or useful increase, in dye uptake will occur.

The treatment can be carried out at temperatures from ambient up to at least 60°C. At ambient temperature, exposure of the material to the complex (or to the separate components of it in a two stage process with or without intermediate drying) for say half an

hour gives adequate results. At 60$^{o}$C, fifteen minutes will suffice, but longer exposure produces better results, and exposures of several hours are not harmful.

Treatment can also be carried out by padding the material with the complex (or with each of the separate components of it with or without intermediate drying in a two stage process) with or without subsequent drying or curing.

The process may be carried out in liquor having a pH value within a wide range. For exhaust application, pH 6-7 is found to be very effective, while for padding, lower pH values can be more effective, e.g. pH 4.

In addition to exhaust and padding application, the process may be carried out by soaking, by spraying, by printing or any other techniques. Two tone dyeing can be produced by applying the treatment to selected areas of a fabric before dyeing. It is preferred not to have too high a liquor/material weight ratio - preferably substantially less than 100:1.

In another method of treatment, the complex, or the chitosan or the complexing compound may be incorporated into a fibre (or sheet or other structure)

as the material is spun or extruded. This is of course akin to the technique referred to above in connection with polypropylene, where dye receptors are incorporated into the melt. However, those dye receptors tend to have a deleterious effect on the other properties of the polypropylene, whereas the chitosan complexes of the present invention do not. It must be ensured that the complex does not degrade, of course, at temperatures required for spinning.

The process may be applied to all usual textile materials, and has been successfully tested on natural fibres such as wool, mohair, silk and cotton, regenerated cellulose fibres such as viscose rayon, cellulose diacetate, cellulose triacetate, synthetic materials such as nylon, polyester, acrylics, polyethylene, polypropylene and polyvinyl chloride, glass fibres and ceramic fibres. The treatment will also be found effective for glass and metals (which may be etched prior to treatment), wood, paper, leather, keratinous materials, synthetic resinous materials and ceramics. In all cases, the treatment can prepare the material for dyeing using any dye with beneficial results compared to the effect of such dye without the pretreatment, but in any event, within the groups of dyes and the groups of materials to cooperate or complex with the chitosan can be found some which will produce

deep shades which are washfast for any one of the materials.

Most importantly, reactive dyes such as Procion dyes (manufactured by Imperial Chemical Industries) and Drimarine dyes (manufactures by Sandoz Chemicals Ltd) can be used after the treatment to give good shades fast to washing and light on all the materials mentioned above. Direct and disperse dyes and fluorescent brightening agents can also be used all on the materials after the treatment with improved washfastness.

The dyeing and printing operations may be carried out as they would be normally, but reduction in the time and temperature of dyeing or the amount of dye used or improvement in the shade obtained may be achieved.

The three Syntan G polar agents referred to above - which are among the compounds that work best with chitosan - may be prepared by the following laboratory methods:

Syntan G1: 0.16 mole of resorcinol is dissolved in 25 ml water by warming. The solution is cooled to room temperature and 0.08 mole of formaldehyde and 0.1 ml of 10% aqueous sulphuric acid solution added.

Gentle heat is applied to reflux and continued at that temperature for 2.5 hours. The product is cooled and neutralised with dilute caustic soda.

Syntan G2: 0.1 mole catechol is stirred in 20 ml water and 2 grams of ammonium chloride. This is heated to 45°C and 0.05 mole formaldehyde added slowly. Heat is applied to reflux and maintained for 2.5 hours. There is no need to neutralise.

Syntan G3: 0.1 mole pyrogallol is stirred in 20 ml water and 2 grams of ammonium chloride. This is heated to 45C and 0.05 mole formaldehyde added slowly. Heat is applied to reflux and maintained for 1.5 hours. There is no need to neutralise.

In addition to the above syntans, other compounds such as commercially available levelling agents, dispersing agents, dye fixing agents and carboxyl methyl cellulose have been found effective, if less so. It should perhaps be noted that the three Syntan G compounds are not typical of commercially available syntans, which tend to have larger molecules and are generally based on aryl sulphonic acid-formaldehyde condensates.

The beneficial effects of the treatment are believed in some aspects to be associated with the formation of a sparingly-soluble complex of large molecular size. This complex will become attached to the various materials with which it is in contact by virtue of ionic forces of attraction operating between the cationic chitosan and anionic groups (carboxylic, hydroxyl, sulphonic acid, etc.) within the material, hydrogen bonding between appropriate groups within the complex and the materials and also van der Waal's forces of attraction operating between the large molecular size complex and the various materials.

The dye must then interact with the chitosan complex and, where appropriate, the material.

It is considered important to rinse the material after the treatment and before the application of colour. The material may be rinsed and then dyed immediately without drying, or it may be dried after rinsing and before dyeing - there seems to be no difference in the result. The importance of rinsing - not carried out in the processes described in GB-A-1 435 229 - is thought to be that the chitosan and its cooperating compound or the chitosan complex which is already inside a fibre, say, attaches to the dye molecule inside the fibre to form a larger structure

that cannot now get out of the fibre. If, however, there is any chitosan or complex on the surface of the fibre, then the larger structure is formed outside the fibre and cannot now get in.

Compounds Syntans G1, G2 and G3 are also found to be useful auxiliaries for colouring materials, for example by dyeing and printing in their own right and without chitosan.

The invention, more generally, comprises a process for colouring materials comprising using as auxiliaries compounds having the form

$$Ring - CH_2 - Ring$$

where the rings have at least one OH group each, the remainder being H or other radicals.

The rings may be benzene, naphthalene or other cyclic structures.

Such compounds have now been found useful as levelling agents and dye resist agents for the dyeing of wool, silk and nylon and for blends containing these fibres, and for improving wash fastness and resistance to chlorinated water of anionic dyes on nylon.

They may be applied to dyed nylon preferably in a low liquor ratio, at preferably low pH values of about 3 - 6.

Wool, nylon and silk can be dyed in the presence of as little as 0.1% of one or other of these compounds.

Resist or reserve styles can be obtained on wool, nylon and silk and blends containing these fibres by treating the undyed material with the said compound by printing, spraying, padding or any other method, followed by normal dyeing and printing operations. Drying may or may not be carried out as an intermediate process.

Many compounds are known to have the property of softening textile fibres, yarns and fabrics and are used in dyeing and finishing. In many cases, the softening agent is specific to a particular material or is best not used with other products intended for the control of or modification to the properties of the material.

The present invention, in another aspect, provides a softening agent which is applicable to all textile materials.

The invention comprises a process for softening textiles comprising applying chitosan as a softening agent.

The invention also comprises applying chitosan to a textile material as a pretreatment to a dyeing process, and washing the material to remove surface chitosan before the dyeing step.

The chitosan will function as a softening agent when applied by padding, by exhaust, by spraying, by soaking or by any other technique, to any undyed, dyed or printed material. In addition, such a pretreatment of undyed material will promote uptake of certain dye classes. for instance, reactive, basic, disperse, acid and direct dyes and fluorescent brightening agents, on selected materials.

However, pretreatment is not essential when applying basic and disperse dyes to appropriate fibres since chitosan can be applied in conjunction with the two named dye classes, when it confers combined softening, levelling and dye uptake promotion effects.

The chitosan may be applied to a basic dye bath, for example, for acrylics, where it might also act as a levelling agent and/or a promoter of basic dyes. The

amount of chitosan may be one or two percent by weight. Lesser and greater amounts, however, may also be used.

In addition, the pretreatment of wool, nylon and silk with chitosan confers both levelling action and dye promotion when these fibres are dyed with appropriate dyes. In printing these fibres, pretreatment with chitosan also confers dye promotion.

Spinning or incorporating chitosan into a fibre (or sheet or other structure) with the material also confers softening on the material as if the chitosan had been incorporated by application to the spun or extruded material.

The amount of chitosan applied may be selected according to choice; the more chitosan is applied the better the effect in terms of softening action, and also in the case of basic and disperse dyes levelling action and dye uptake promotion action, although a limit to the amount of chitosan applied will occur according to the type of material used, beyond which no further increase in desired properties will occur.

The treatment may be carried out at temperatures from ambient up to at least 60$^{o}$C. At ambient temperature exposure of the material to chitosan for,

say, thirty minutes gives adequate results. At higher temperatures, shorter exposure to chitosan will suffice. Prolonged exposure of material to chitosan is not harmful.

The treatment may be carried out in a liquor having a pH value over a wide range but preferably at pH 6.

Complexes of chitosan with the complexing agents referred to above will also act as softening agents for textile materials, and they may be applied in a single step or in a two stage process in which the material is treated with chitosan and then the complexing agent, or the other way round, the complex being formed in or on the material. The treatment can be carried out by the same methods and under the same conditions as for chitosan on its own.

An aftertreatment with such complexes will, in addition to imparting the above stated effects, also improve the washfastness and resistance to chlorinated water of acid dyes on nylon.

Methods and products for treating and colouring materials in accordance with the various aspects of the invention will now be further described with reference

to the following Examples:

Example 1: Polypropylene yarn was pretreated with 2% on weight of fibre ("o.w.f.") of a chitosan complex consisting of a 1:1 mixture of chitosan and Syntan Gl in tap water (pH 8) at 50:1 liquor ratio. The polypropylene was immersed in the liquor for 15 minutes at 60°C. The yarn was then removed from the liquor and rinsed thoroughly in tap water.

A fresh bath was made up (10:1 liquor ratio) containing 2% o.w.f. Procion U.C. red MX-5B dyestuff and 30g/l sodium chloride. The pretreated yarn was immersed in the bath and agitated for 10 minutes at 40°C, then 5g/l sodium carbonate was added and the bath further agitated for 60 minutes at 40°C.

The yarn was thoroughly rinsed and then dried.

The polypropylene yarn was found to be dyed to a good red shade and had good washfastness.

Example 2: A cotton fabric was pretreated with 2% o.w.f. of a chitosan complex consisting of a 1:1 mixture of chitosan and ammonium sulphate.

The fabric was immersed in a liquor of tap water

(pH 8) containing the complex (liquor ratio 50:1) and agitated for 15 minutes at 60°C. The fabric was then thoroughly rinsed in tap water.

A fresh bath was made up containing 2% o.w.f. Procion U.C. navy HER 150 dyestuff (liquor ratio 50:1). The fabric was agitated in the bath for 10 minutes at 60°C and then 60g/l sodium chloride was added and the bath further agitated for 15 minutes at 80°C. Then 15g/l sodium carbonate was added and the bath further agitated at 80°C for one hour.

The dyed cotton fabric was then thoroughly rinsed and boiled for 2 minutes at 100°C, then dried.

The fabric was dyed a deep navy and had good washfastness.

Example 3: An acrylic fabric was pretreated with 0.5% o.w.f. of a chitosan complex comprising a 1:1 mixture of chitosan and Cibatex PA (a sulphonated polyphenol, an anionic fixing agent or synthetic tanning agent manufactured by Ciba-Geigy). The fabric was padded with the mixture to give a pick-up of 100% and was then dried at 80°C for four hours. The fabric was thoroughly rinsed and then dyed by immersion in a bath of 50:1 liquor ratio at 60°C containing 1% o.w.f. of

Nylomine Red A-2B (Sandoz Products Limited), 3% o.w.f. ammonium acetate and sufficient acetic acid to give a pH of 4.5. The dye bath was raised to the boil over 30 minutes and then boiled for one hour. The fabric was then thoroughly rinsed.

On drying, the fabric was dyed to a good red shade and was washfast.

Example 4: A nylon fabric was pretreated with a complex of chitosan with a complexing compound formed from diphenyl oxide, sulphuric acid, formaldehyde and salicylic acid together with glycerol and then thoroughly washed. Dyeing was carried out by immersing the fabric in a bath of 50:1 liquor ratio at 60$^{o}$C containing 1% o.w.f. of Maxilon M-2G (Ciba Geigy) adjusted to pH 4.5 with a mixture of sodium acetate and acetic acid. The bath was raised to the boil over 30 minutes and boiled for one hour. The fabric was then thoroughly rinsed.

The nylon fabric was dyed a strong blue colour and had very good washfastness.

Example 5: A wool fabric was pretreated in two steps:

Step 1 - the fabric was padded with a 0.5% chitosan

solution at pH 4 at a pick up of 100%. The fabric was then dried at 80°C for 4 hours or alternatively cured at 160°C for 3 minutes.

Step 2 - the fabric was then rinsed thoroughly and then sprayed with a glycine solution to approximately a 0.5% treatment. The fabric was redried.

The dyeing was carried out using 2% o.w f. Diphenyl Red 5B (182%) and 22% o.w.f. sodium chloride and was carried on for one and a half hours at 80°C. The fabric was thoroughly rinsed. The wool fabric dyed more deeply than the same fabric not pretreated.

Example 6: A cellulose diacetate fabric was pretreated with a 1:1 chitosan - ammonium sulphate complex as in Example 2, and rinsed thoroughly.

A fresh bath was then made up (50:1 liquor ratio) containing a 2% o.w.f. optical brightening agent (Uvitex TC665) the pH being preadjusted to pH 5 with acetic acid. The bath was agitated for 40 minutes at 95C and the fabric thoroughly rinsed afterwards.

Example 7: A cotton/polyester fabric was pretreated as in Example 2 only with Ampholan B171 in place of the ammonium sulphate. The fabric was dyed as in Example 1

only with Procion Blue MX-2GN.

Example 8: A polyester/cotton fabric was pretreated as in Example 1, but with Gloquat C in place of Syntan Gl. The fabric was dyed as in Example 2.

The polyester/cotton fabric dyed more deeply than the same fabric not pretreated. A fabric similarly pretreated with chitosan alone, dyed more deeply than the same fabric not pretreated, but less deeply than the same fabric pretreated with chitosan and Gloquat C.

Example 9: Cotton and polyester/cotton fabrics are pretreated in two steps:

Step 1 - the fabrics are sprayed with a 100% (W/V) solution of sodium lauryl sulphate to give an approximate pick-up of 50%. The fabrics were then dried at 80°C.

Step 2 - the above treated fabrics were padded with 0.5% (W/V) solution of chitosan to give a pick-up of 98%. Drying for one hour at 80°C then followed.

These pretreated fabrics are rinsed and then dyed as is Example 7, when dyeings of deeper shades than the same fabrics not pretreated, are obtained.

Example 10: Polyester tow was pretreated as in Example 1, and dyed also as in Example 1.

A red shade of good light and washfastness was obtained.

Among anionic compounds found useful for complexing with chitosan to give good, washfast colour yields with reactive dyes on all materials tested are products of the following:

phenol + sulphuric acid + formaldehyde
o-phenyl phenol + sulphuric acid + formaldehyde + salicylic acid
4, 4' dihydroxydiphenyl sulphone + salicylic acid + formaldehyde, in the presence of either sulphuric acid or sodium hydroxide
diphenyl oxide + sulphuric acid + formaldehyde + salicylic acid

These are essentially products of condensation of formaldehyde with one or more components of the type which are hydroxy-containing ring structures which may or may not be carboxylated or sulphonated, which may or may not contain a sulphone bridge, and which may have further sulphonation after condensation. The condensation reactions may be carried out in an acidic

medium as provided by, for example, sulphuric acid, hydrochloric acid or ammonium chloride, or in an alkaline medium as provided by, for example, sodium hydroxide. Higher bi-functional aldehydes may be used instead of formaldehyde.

Among proprietary products which are anionic complexing agents are sodium lauryl sulphate (which must be used in a two stage process, with the chitosan being applied either first or second), Cibatex PA (a product of Ciba-Geigy) and Nylofixan PE liquid (a product of Sandoz).

A proprietary amphoteric compound is Ampholan B171, a product of Lankro Chemicals, which is an amphoteric wetting agent or surfactant. Amino acids such as lycine may also be used.

Neutral electrolytes or salts such as sulphates, chlorides, carbonates and bicarbonates of ammonium, sodium and potassium give good results when used with chitosan as pretreatment for reactive dyes, and show some improvement with acid dyes.

Polar or non-ionic compounds include the products of condensation of formaldehyde and higher bi-functional aldehydes with one or more components of

the type which are hydroxy-containing ring structures, but which are not carboxylated or sulphonated. Ethoxylated phenols are also found useful.

Gloquat C - a quaternary ammonium compound obtainable from ABM Chemicals - is a cationic compound which can also be used with chitosan. Since chitosan is also cationic, it may not complex with Gloquat C as it does with the other compounds referred to, but the two may be used together or one applied first and the other afterwards with or without intermediate drying. Generally, any aryl, alkyl or aryl-alkyl quaternary ammonium compounds, including particularly chlorides and bromides will be found useful cationic compounds.

CLAIMS:

1 A process for preparing a material for coloration by dyeing, printing and other methods and for softening the material where appropriate characterised by treating the material with a treatment compound selected from the group consisting of an amino sugar, a ring compound of the form

$$\text{Ring} - CH_2 - \text{Ring}$$

a complex of an amino sugar with such a ring compound, a complex of an amino sugar with a complexing compound selected from the group consisting of an anionic compound, an amphoteric compound, a non-ionic compound, a polar compound and a neutral electrolyte, and an amino sugar in the presence of a cationic compound.

2 A process according to claim 1, wherein the material is a textile material and is rinsed following the treatment and before colouring to remove treatment compound from the surface.

3 A process according to claim 1 or claim 2, wherein the amino sugar comprises chitosan.

4 A process according to any one of claims 1 to 3, wherein the amino sugar and the complexing compound are applied in separate steps.

5 A process according to any one of claims 1 to 4, of which at least a part is carried out by a wet process.

6 A process according to claim 5, in which the said wet process is selected from the group comprising spraying, padding, soaking, printing, and an exhaust process.

7 A process according to any one of claims 1 to 6, in which the said treatment compound is incorporated in an extrudate.

8 A process according to any one of claims 1 to 7, comprising treating the material with chitosan and at least one compound selected from the group of cationic compounds consisting of aryl, alkyl and aryl-alkyl quaternary ammonium compounds.

9 A process according to any one of claims 1 to 8, comprising treating the material with chitosan and at least one compound selected from the group of anionic compounds consisting of products of condensation of formaldehyde and higher bi-functional aldehydes with one

or more components of the type which are hydroxy-containing ring structures, such compounds which contain a sulphone bridge, such compounds which are carboxylated, such compounds which are sulphonated, such compounds which are catalysed by an acid, such compounds which are catalysed by a base and such condensation, products on which sulphonation is carried out after condensation.

10 A process according to any one of claims 1 to 9, comprising treating the material with chitosan and sodium lauryl sulphate in a two stage process.

11 A process according to any one of claims 1 to 10, comprising treating the material with chitosan and a condensation product of 4, 4'-dihydroxydiphenyl sulphone and formaldehyde.

12 A process according to any one of claims 1 to 11, comprising treating the material with chitosan and an amphoteric wetting agent.

13 A process according to any one of claims 1 to 12, comprising treating the material with chitosan and an amino acid.

14 A process according to any one of claims 1

to 13, wherein the ring compound is a polar compound which is a synthetic tanning agent selected from the group consisting of Syntans G1, G2 and G3.

15 A process according to any one of claims 1 to 14, comprising treating the material with chitosan and a polar and non-ionic compound which is an ethoxylated phenol.

16 A process according to any one of claims 1 to 15, comprising treating the materials with chitosan and a neutral electrolyte selected from the group consisting of sulphates, chlorides, and carbonates of sodium, potassium and ammonium.

17 Complexes of an amino sugar and at least one complexing compound selected from the group consisting of anionic compounds, amphoteric compounds, non-ionic compounds, polar compounds and neutral electrolytes, useful in preparing materials for colouring and where appropriate softening the same.

18 Ring compounds for use in a process according to any one of claims 1 to 16, having the form:

$$\text{ring} - CH_2 - \text{ring}$$

where the rings have at least one OH group each, the remainder being selected from H and other radicals.

19 Ring compounds according to claim 18, wherein the rings are benzene rings.

20 Ring compounds according to claim 18, wherein the rings are naphthalene rings.

21 A compound having the formula:-

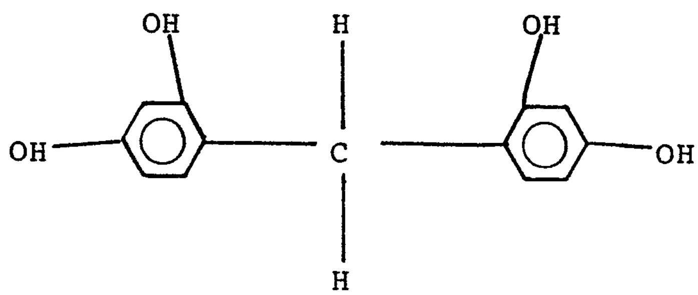

Syntan G1

22 A compound having the formula:-

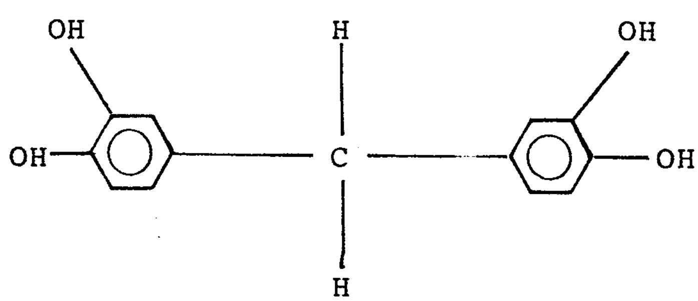

Syntan G2

23 A compound having the formula:-

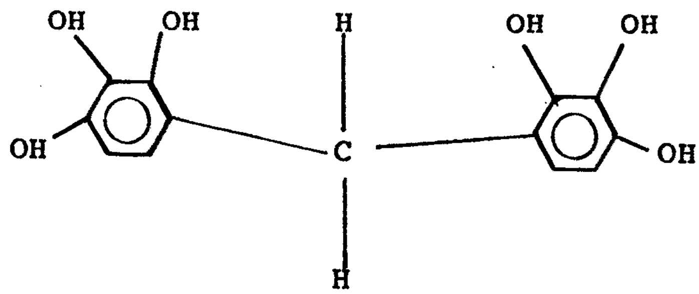

Syntan G3